# DEMANDE DE BREVET EUROPEEN

(11) **EP 3 031 477 A1**
(43) Date de publication de la demande: **15.06.2016**
(21) Numéro de dépôt: 15199117.1
(22) Date de dépôt: 10.12.2015
(51) Int. Cl.: A61L 15/50, A61L 15/58

(54) **INTERFACE CICATRISANTE NON ADHÉRENTE, COMPRESSE ET PANSEMENT ASSOCIÉS**

(30) Priorité: 11.12.2014 FR 1462248
(71) Demandeur: Gergonne SAS, 01100 Oyonnax (FR)
(72) Inventeur: GERGONNE, Charles, 01100 ARBENT (FR); GERGONNE, Bertrand, 01100 ARBENT (FR); ROBÉ, Frédéric, 21700 AGENCOURT (FR)
(74) Mandataire: Agasse, Stéphane

(57) **Abrégé**

La présente invention concerne une interface cicatrisante non adhérente (10) comportant au moins un filet (2) à mailles ouvertes et qui se caractérise en ce que le filet (2) a été enduit d'un mélange (3) comprenant au moins du gel de silicone et au moins un agent gélifiant.

La présente invention a aussi pour objet une compresse (12) et un pansement (1) qui comprennent au moins ladite interface (10).

## Description

La présente invention concerne une interface cicatrisante non adhérente stérile destinée à être appliquée directement au contact d'une plaie, ainsi qu'une compresse et un pansement comprenant une telle interface.

Parmi les dispositifs médicaux, il existe des interfaces cicatrisantes non adhérentes qui sont destinées à être appliquées directement sur une plaie en vue de la cicatriser, voire d'accélérer le processus naturel de cicatrisation.

De telles interfaces sont souvent conçues pour être destinées à être appliquées sur des plaies aiguës, chroniques, et parfois très exsudatives de personnes affaiblies (par exemple des grands brûlés, des personnes en fin de vie).

Ainsi, il est essentiel que les interfaces soient conçues pour créer les conditions optimales nécessaires au processus de cicatrisation tout en étant le moins possible traumatique pour le patient, lors de la pose et surtout lors du retrait de l'interface.

Les interfaces cicatrisantes non adhérentes connues de l'état de l'art ne sont pas pleinement satisfaisantes pour les raisons suivantes :
- leur pose et leur maintien sur la plaie ne sont pas toujours aisés du fait qu'elles ne présentent pas toujours des propriétés adhésives immédiates adéquates,
- leur retrait de la plaie peut être traumatique,
- la cicatrisation régulière et rapide de la plaie n'est pas toujours obtenue, ou alors très difficilement.

Il est connu que la cicatrisation de la plaie ne peut évoluer favorablement que si l'interface n'adhère pas aux tissus nouvellement régénérés et que si les exsudats sont éliminés tout en créant un environnement humide autour et sur la plaie.

C'est pourquoi, il serait souhaitable de disposer d'une interface qui soit parfaitement anti-adhérente sur les tissus régénérés, et qui crée et maintienne des conditions d'humidité optimales favorables à la cicatrisation, tout en évitant le risque de macération.

La présente invention remplit parfaitement ces objectifs détaillés ci-dessus en proposant une nouvelle interface cicatrisante non adhérente qui comporte au moins un filet à mailles ouvertes et qui se caractérise en ce que le filet a été enduit d'un mélange comprenant au moins du gel de silicone et au moins un agent gélifiant.

De manière préférée, au moins une partie desdites mailles ne sont pas obstruées par ledit mélange comprenant au moins du gel de silicone et au moins un agent gélifiant.

Dans un mode de réalisation de l'invention, une majorité desdites mailles ne sont pas obstruées par ledit mélange comprenant au moins du gel de silicone et un agent gélifiant.

Dans un mode de réalisation de l'invention, aucune maille du filet n'est obstruée par ledit mélange.

Le mélange dont est enduit le filet de l'interface, comprend essentiellement du gel de silicone. De préférence, le poids en gel de silicone représente au moins 80%, de préférence de 80% à 95%, du poids total dudit mélange.

Le gel de silicone est connu et approprié pour faire adhérer temporairement un dispositif médical à la peau. Le gel de silicone procure une adhérence élevée à la peau et une faible force de libération par décollement. De préférence, le pouvoir adhésif du gel de silicone que comprend ledit mélange est compris entre 0,5 N/cm et 2 N/cm.

Dans le cadre de la présente invention, le gel de silicone que contient le mélange enduit sur le filet de l'interface est sous forme réticulée. Il présente donc une certaine cohésion.

Dans le cadre de la présente invention, on entend par agent gélifiant, un composé qui forme un gel en milieu aqueux, par exemple dans de l'eau.

L'interface selon l'invention présente l'avantage d'adhérer légèrement sur la plaie du fait que le gel de silicone dudit mélange dont est enduit le filet est légèrement adhésif. Cette propriété facilite la mise en place de l'interface sur la plaie du patient et permet, éventuellement, de la replacer aisément si nécessaire.

En outre, du fait des mailles ouvertes que comporte le filet qui sont de préférence en majorité non obstruées par le mélange de gel de silicone et d'agent gélifiant, les exsudats de la plaie sont évacués au fur et à mesure de la cicatrisation, et lors de leur passage au travers les mailles du filet, l'agent gélifiant contenu dans ledit mélange va s'hydrater et former un gel. En d'autres termes, en traversant le filet enduit d'un mélange comprenant essentiellement du gel de silicone et au moins un agent gélifiant, une partie des exsudats est captée par le caractère hydrophile de l'agent gélifiant. Celui-ci se transforme alors progressivement en gel humide en :
- créant ainsi un environnement autour de la plaie qui est favorable pour la cicatrisation des plaies, et même des plaies difficiles, de telle sorte que la cicatrisation de la plaie soit partielle, voire totale,
- rendant l'interface parfaitement atraumatique lors de son retrait. En effet, l'agent gélifiant transformé en gel tend à diminuer les propriétés adhésives précitées du gel de silicone et rend ainsi l'interface selon l'invention parfaitement anti-adhérente; ce qui préserve l'intégrité des nouveaux bourgeons cicatriciels qui sont les plus fragiles.

Ainsi, l'association du gel de silicone et de l'agent gélifiant confère à l'interface selon l'invention des propriétés extrêmement favorables à la cicatrisation :
- le gel de silicone donne une bonne stabilité physique à l'interface qui peut rester en place sur la plaie plusieurs jours, sans migrer ou se disperser et sans adhérer aux tissus fraîchement régénérés, tout en permettant une liaison avec un large choix de matériaux absorbants tels qu'une mousse de polyuréthane, et ce comme cela sera expliqué ci-après,
- l'agent gélifiant qui se transforme en gel au fur et à mesure de l'évacuation des exsudats permet de maintenir un degré d'humidité favorable au processus de cicatrisation, en évitant un dessèchement de la plaie et, en conséquence, soit la formation d'une croûte, soit une adhérence de l'interface sur la plaie.

L'interface cicatrisante non adhérente selon l'invention comprend un filet à mailles ouvertes.

Le filet est avantageusement formé à partir de fils qui peuvent être réalisés en un matériau flexible, peu extensible ou encore non élastique. Ledit filet peut avoir été obtenu par tout procédé de tissage ou de tricotage qui est parfaitement à la portée de l'homme du métier.

Le filet peut être parfaitement rigide (autrement dit les mailles sont bloquées) ou bien légèrement extensible (avec une extensibilité comprise avantageusement entre 20% et 50%) dans le sens chaîne et/ou le sens trame.

De manière préférée, les diamètres des fils sont les mêmes en chaîne et en trame afin de permettre la régularité de l'enduction du mélange qui comprend essentiellement du gel de silicone.

Dans le mode de réalisation où le filet a été obtenu par tissage, les mailles peuvent être fixées au moyen de fils de tour afin d'obtenir une bonne stabilité dimensionnelle.

Dans un mode de réalisation de l'invention, le fil utilisé pour fabriquer le filet est préférentiellement un fil continu à filaments, très peu extensible et non élastique, l'extensibilité étant de préférence inférieure à 35 %.

Par fil continu à filaments, on entend un fil formé de un ou plusieurs filaments longs retords. Le choix de filaments longs permet d'éviter les fibres courtes qui risquent de se détacher du filet et venir se disperser près de la surface de contact avec la plaie.

Dans un mode de réalisation de l'invention, le matériau des fils du filet est un matériau synthétique. De préférence, il s'agit d'un matériau hydrophobe.

Ainsi, le matériau des fils du filet peut être choisi parmi les polyesters (par exemple les polyéthyltéréphtalates), les polypropylènes, les polyéthylènes, les polyamides ou encore les acétates de cellulose. Ces différents matériaux présentent l'avantage de fournir des filaments longs et des fils présentant beaucoup moins de fibrilles que les fils obtenus à partir de fibres courtes.

Le choix de certains matériaux tels que les polyesters permet aussi de thermo-fixer la structure à mailles ouvertes du filet. Ainsi, selon un mode de réalisation de l'invention, le filet est formé à partir de fils continus de polyester qui ont été thermo-fixés pour former des mailles. Un tel filet présente les avantages d'être pratiquement non extensible dans les directions chaîne ou trame et de se travailler plus facilement que d'autres filets élastiques. Une telle structure du filet garantit une enduction régulière des fils du filet par le mélange qui, rappelons-le comprend essentiellement du gel de silicone. Ainsi, lorsque les fils du filet sont formés de polyesters, cela présente l'avantage que l'enduction par le mélange est facilitée.

De manière préférée, les fils du filet sont en polypropylène et/ou en polyéthylène.

Dans un mode de réalisation de l'invention, le filet à mailles ouvertes est réalisé avec des fils constitués d'un même matériau, par exemple un des matériaux décrits ci-dessus. Dans un autre mode de réalisation de l'invention, le filet à mailles ouvertes est réalisé avec des fils de chaîne et des fils de trame qui sont constitués de matériaux différents.

De manière préférée, les mailles ouvertes du filet sont larges, de préférence de taille régulière et présentent une forme générale, par exemple, ronde, ovale, ou encore polygonale (telle que rectangulaire, carrée). De manière avantageuse, les mailles ont des ouvertures comprises entre 1 et 5 mm de côté. La dimension des mailles est telle que la surface unitaire des ouvertures est comprise entre 0,5 et 10 mm², préférentiellement de 0,5 à 3 mm². Par exemple, il peut s'agir de mailles dont l'ouverture correspond à environ 4 à 20 mailles par cm.

Le taux d'ouverture du filet (à savoir le rapport de la surface ouverte du filet sur la surface totale dudit filet) est avantageusement compris entre 50 et 90 %.

Une fois le filet enduit avec le mélange contenant au moins du gel de silicone et l'agent gélifiant, le taux d'ouverture du filet est préférentiellement compris entre 40 % et 90 %, plus préférentiellement entre 75 % et 90 %.

Dans un mode de réalisation de l'invention, le taux de perméabilité à la vapeur d'eau de l'interface selon l'invention est compris entre 50 et 90 g/m²/24 heures, de préférence entre 75 et 90 g/m²/24 heures.

De manière particulièrement avantageuse, le filet a été, préalablement à l'enduction, lavé pour éliminer toutes traces d'enzymages ou de résidus d'un traitement préalable auquel auraient été soumis les fils du filet.

Le filet de l'interface a été enduit d'un mélange qui comprend essentiellement du gel de silicone.

Le gel de silicone, composé hydrophobe, stable en milieu humide, permet de bien emprisonner les fils du filet qui restent parfaitement isolés de la plaie aussi longtemps que l'interface selon l'invention reste en place sur la peau. Ainsi, il n'y a pas de risque d'un contact direct entre les fils du filet et les tissus régénérés, ce qui pourrait provoquer une inclusion des fils du filet dans la plaie, avec pour conséquence une destruction douloureuse des tissus au moment du retrait de l'interface.

De manière préférée, le gel de silicone est un mélange d'une résine de poly-di-organosiloxane et de catalyseur, par exemple dans un ratio en poids de ces composés précités de 1: 1, 3 : 1 ou encore de 10 : 1, ledit mélange de poly-di-organosiloxane et de catalyseur ayant été soumis à une réticulation comme cela sera expliqué plus en détail ci-après dans la description de la fabrication de l'interface ou d'une compresse selon l'invention. Le gel de silicone utilisé dans le cadre de la présente invention peut être celui décrit dans le brevet US 4 991574.

De préférence, le gel de silicone est choisi parmi les gels de silicone parfaitement compatibles avec la peau, de préférence avec de faibles viscosités et des durées de vie de pot ouvert supérieures à 30 minutes.

Préférentiellement, le gel de silicone est choisi parmi les produits de :
- la société WACKER qui sont dénommés SILPURAN, séries 2110 et 2120,
- la société BlueStar Silicones International de la série SILBIONE 4717 ou encore
- la société NUSIL qui sont dénommés CEREPLAST MED 634.

Le procédé de réticulation du gel de silicone est parfaitement à la portée de l'homme du métier.

Le mélange dont a été enduit le filet de l'interface comprend en outre au moins un agent gélifiant.

L'agent gélifiant a été avantageusement dispersé de manière homogène dans le mélange dont est enduit le filet de l'interface selon l'invention, et ce préférentiellement en quantité relativement faible de manière à ce que ledit mélange comprenant essentiellement du gel de silicone (à savoir un composé hydrophobe) présente un caractère légèrement hydrophile, suffisant pour maintenir l'environnement humide favorable à la cicatrisation qui a été mentionné ci-dessus et qui empêche un dessèchement de la plaie qui pourrait conduire à une adhérence de l'interface, mais insuffisant pour rendre le gel de silicone capable d'absorber beaucoup d'eau. En effet, ce pouvoir absorbant du gel de silicone n'est pas souhaitable car il entraînerait un gonflement du mélange dont est enduit le filet de l'interface ; ce qui provoquerait une obstruction progressive des mailles du filet.

Préférentiellement, le pourcentage massique de l'agent gélifiant dans le mélange dont est enduit le filet est compris entre 0,1% et 20%, de préférence entre 5% et 15%, encore plus préférentiellement entre 8% et 10%.

Des quantités supérieures d'agent gélifiant dans le mélange pourraient perturber le caractère cohésif du gel de silicone du mélange ; ce qui accentuerait exagérément le caractère hydrophile et modifierait ainsi trop considérablement les propriétés légèrement adhésives que présente l'interface selon l'invention grâce au gel de silicone.

De manière avantageuse, l'agent gélifiant est choisi parmi :
- les celluloses et leurs dérivés tels que l'hydroxypropylméthylcellulose, l'hydroxypropyléthylcellulose, la méthylcellulose, la carboxyméthylcellulose et l'hydroxyéthylcellulose, l'éthylcellulose, l'éthylhydroxyethylcellulose, l'hydroxypropylcellulose,
- les alginates tels que l'alginate de sodium,
- l'acide alginique,
- l'acide polyacrylique,
- les gommes naturelles telles que la gomme de xanthane, adragante ou encore de guar,
- l'alcool polyvinylique, les polyvinylpyrrolidones et leurs dérivés,
- les copolymères des polyoxyéthylènes et des polyoxypropylènes,
- le chitosane,
- les pectines.

De manière tout à fait préférée, l'agent gélifiant est la carboxyméthylcellulose (abrégée ci-après « CMC »). En effet, la CMC présente l'avantage de pouvoir absorber jusqu'à 10 fois son poids.

Dans un mode réalisation de l'invention, le mélange en poids comprend de 5% à 15% de CMC; ce qui permet d'obtenir un mélange qui, après son enduction sur un filet, présente un état de surface restant légèrement adhésif et absorbant au contact d'une plaie.

De préférence, la CMC se présente sous la forme de particules. Les particules de CMC sont maintenues dispersées dans le mélange grâce à la cohésion du gel de silicone qui permet leur hydratation et leur transformation en gel. En se transformant en gel, les fibres de CMC hydratent les fibres voisines qui à leur tour se transforment en gel et ainsi de suite.

Dans un mode de réalisation de l'invention, l'agent gélifiant se présente sous forme d'une poudre fine. De manière avantageuse, l'agent gélifiant est sous forme solide et finement divisé, par exemple sous la forme d'une poudre dont la granulométrie moyenne peut être inférieure à 100 µm, de préférence inférieure à 50 µm. Le gel de silicone permet une dispersion homogène des particules d'agent gélifiant.

Selon un mode de réalisation de l'invention, l'agent gélifiant, de préférence sous la forme d'une poudre fine, a été ajouté au gel de silicone dans un mélangeur qui a été soumis à un malaxage, de manière à ce que sa dispersion soit homogène dans le gel de silicone. Le mélange intime du gel de silicone et de l'agent gélifiant permet d'obtenir un mélange capable de maintenir un environnement humide propice à la cicatrisation comme cela a été détaillé ci-dessus.

Dans un mode de réalisation de l'invention, le mélange dont est enduit le filet de l'interface comprend en outre au moins un principe actif, par exemple :
- un principe actif présentant un effet thérapeutique destiné à améliorer les propriétés cicatrisantes de l'interface, par exemple l'acide salicylique ou l'acide hyaluronique,
- un principe actif présentant des propriétés antiseptiques, par exemple la sulfadiazine argentique, le chlorure de benzalkonium ou les sels d'argent,
- des antibiotiques comme par exemple la néomycine ou la polymyxine,
- des anti-inflammatoires comme par exemple l'acétonide de triamcinolone.

De manière avantageuse, le mélange comprend en poids entre 1% et 10%, de préférence entre 1% et 5%, de principe actif.

Dans une mode de réalisation préféré de l'invention, le mélange comprend entre 1% et 5% en poids d'acide hyaluronique.

Le choix et la quantité des principes actifs qui sont incorporés dans le mélange qui comprend essentiellement du gel de silicone sont parfaitement à la portée de l'homme du métier.

Le filet de l'interface selon l'invention a été enduit d'un mélange qui comprend essentiellement du gel de silicone. L'enduction a été réalisée de façon à laisser non obstruées une majorité des mailles du filet tout en enrobant (ou autrement dit en emprisonnant) les fils du filet avec le mélange.

La quantité de mélange dont sont enduits les fils du filet va dépendre de la structure du filet, à savoir de l'ouverture de ses mailles.

Dans un mode de réalisation de l'invention, la quantité du mélange qui comprend essentiellement du gel de silicone est comprise entre environ 50 et 300 g/m², de préférence entre environ 100 et 300 g/m², plus préférentiellement entre environ 120 et 200 g/m² de l'interface.

L'interface selon l'invention peut être fabriquée de la manière suivante.

Comme cela a été expliqué ci-dessus, le gel de silicone est un mélange de résine de poly-di-organosiloxane et de catalyseur qui a été soumis à une réticulation.

Plus précisément, ce mélange de résine de poly-di-organosiloxane et de catalyseur qui est soumis à une réticulation résulte du mélange d'une 1^{ière} composition comprenant ladite résine de poly-di-organosiloxane et le catalyseur en des quantités déterminées avec une 2^{ième} composition comprenant ladite résine de poly-di-organosiloxane en une quantité déterminée et qui est dépourvue de catalyseur.

La réticulation du gel de silicone (à savoir la polymérisation du gel de silicone) est amorcée dès le mélange de la 1^{ière} composition avec la 2^{ième} composition (autrement dit dès la mise en contact de la 1^{ière} composition avec la 2^{ième} composition), et ce du fait du choix des quantités déterminées des constituants de la 1^{ière} et 2^{ième} compositions pour que la réticulation débute.

Le choix et les quantités des constituants de la 1^{ière} et 2^{ième} compositions en vue d'obtenir un gel de silicone réticulé sont parfaitement à la portée de l'homme du métier. Dès la réunion de la 1^{ière} et 2^{ième} compositions, la réticulation est amorcée. Ensuite, une étape de réticulation dont les conditions sont détaillées ci-après est mise en oeuvre.

Le procédé de fabrication de l'interface selon l'invention peut comprendre les étapes suivantes :
a) On prépare un 1^{ier} mélange préparatoire comprenant au moins un agent gélifiant et éventuellement au moins un principe actif tels que décrits ci-dessus. De préférence, l'agent gélifiant et les éventuels principes actifs se présentent sous la forme d'une poudre.
b) Ensuite, on incorpore ce 1^{ier} mélange préparatoire dans une 1^{ière} composition qui comprend au moins une résine de poly-di-organosiloxane et un catalyseur en des quantités déterminées. Cette incorporation de ce 1^{ier} mélange préparatoire est réalisée de manière régulière, par exemple pendant une durée d'environ 1 minute, dans un mélangeur en marche à faible vitesse. Dans un mode de réalisation, le mélangeur est un mélangeur de type planétaire et la rotation de l'hélice est comprise entre 50 et 100 tours/minute. A l'issue de cette incorporation de ce 1^{ier} mélange préparatoire dans la 1^{ière} composition, on obtient un 2^{ième} mélange préparatoire.
c) Ensuite, on incorpore dans ce 2^{ième} mélange préparatoire ainsi obtenu une 2^{ième} composition qui comprend au moins une quantité déterminée de la résine de poly-di-organosiloxane et qui est dépourvue de catalyseur.
d) On mélange l'ensemble ainsi obtenu à l'étape c) à faible vitesse, par exemple avec une rotation de l'hélice qui est comprise entre 50 et 100 tours/minute, et ce jusqu'à l'obtention d'un mélange homogène qui comprend essentiellement du gel de silicone.
e) On enduit le mélange obtenu à l'étape d) sur les fils d'un filet.
f) On réticule le gel de silicone que comprend le mélange.

Les étapes a) à d) décrites ci-dessus peuvent aussi bien être réalisées en continu qu'en discontinu (autrement dit en « mode batch »).

Du fait du mélange de la 1^{ière} composition et de la 2^{ième} composition précitées, la réticulation du gel de silicone est amorcée au cours de cette étape c) et l'on obtient un mélange à l'étape d) qui comprend essentiellement du gel de silicone.

De manière avantageuse, au cours de l'étape c) d'incorporation dans le 2^{ième} mélange préparatoire de la 2^{ième} composition, on effectue un dégazage des bulles d'air contenues dans ce mélange pour améliorer la cohésion du gel de silicone après réticulation. Dans un mode de réalisation de l'invention, ledit dégazage est réalisé en mettant le mélange en dépression, par exemple entre 0,5 et 0,1 bar.

Le mélange ainsi obtenu à l'étape d) présente une viscosité élevée, de préférence comprise entre 10 000 et 100 000 cps. C'est pourquoi, le procédé d'enduction du filet est adapté pour prendre en compte cette viscosité élevée dudit mélange.

De manière avantageuse, le filet se présente sous la forme d'une bande.

L'enduction du mélange sur le filet peut être réalisée selon les étapes telles que détaillées ci-dessous, et ce par exemple dans une installation d'enduction. De manière préférée, l'installation d'enduction présente des éléments appropriés qui sont parfaitement à la portée de l'homme du métier pour enduire le filet qui se présente sous la forme d'une bande.

Tout d'abord, on trempe intégralement le filet de l'interface dans ledit mélange dont la préparation a été décrite ci-dessus.

Le filet se charge alors d'un excédent dudit mélange. On lamine progressivement le filet pour l'acheminer ensuite sous un premier racle d'enduction qui pré-régule le grammage du mélange déposé sur le filet. Ensuite, on achemine ledit filet sous un deuxième racle d'enduction pour réguler finement le grammage final du mélange déposé sur ledit filet.

Dans un autre mode de réalisation de l'invention, on fait passer le filet dans un bain comprenant le mélange tel que décrit ci-dessus. Le filet recouvert dudit mélange est ensuite passé entre deux cylindres rotatifs pressés l'un contre l'autre avec un écartement prédéterminé de façon à éliminer l'excèdent de mélange. La quantité de mélange restant sur les fils du filet dépend essentiellement de l'écartement imposé entre les cylindres fixes.

De manière à assurer une perméabilité optimale du filet après l'enduction, on effectue un soufflage sur le filet ainsi enduit du mélange uniquement au niveau des fils, par exemple dans une zone de ventilation verticale de manière à ce que le mélange ne coule pas et demeure régulièrement enrobé autour des fils et que l'aération de la grille soit parfaitement maîtrisée.

On peut aussi appliquer un jet d'air sur le filet après le passage sous le racle d'enduction ou entre les deux cylindres rotatifs pressés l'un contre l'autre.

Dans un mode de réalisation de l'invention, après l'étape e) d'enduction, l'aération régulière du filet de manière à ce que les mailles du filet demeurent ouvertes après l'enduction est obtenue par soufflage d'air et/ou par le passage du filet sur un cylindre en inox de telle sorte que le mélange en excédent reste collé sur le cylindre en inox. Préférentiellement, on racle ensuite ce cylindre en inox de manière à le nettoyer en continu et à recycler le mélange en excédent.

Dans un mode de réalisation de l'invention, un système à jet d'air laminaire peut être intégré dans l'installation d'enduction pour corriger la répartition du mélange autour des fils et déboucher les ouvertures de mailles qui n'auraient pas été ouvertes lors du passage dans le deuxième cylindre.

Avec ce procédé d'enduction tel que décrit ci-dessus, les grammages dudit mélange enduit sur le filet sont avantageusement compris entre 100 g/m² et 300 g/m². Cela correspond à une épaisseur d'enduction dudit mélange comprise entre 200 et 600 µm. Ainsi, l'ensemble des mailles du filet est enduit de mélange et on recouvre environ 50% de la surface disponible avec du mélange, les autres 50% de la surface restent parfaitement ouverts et perméables aux exsudats de la plaie et à l'air.

Selon un autre mode de réalisation envisageable de l'invention, le gel de silicone comprenant éventuellement des principes actifs a été enduit sur le filet de façon à enrober les fils et à laisser les ouvertures de mailles non obturées, par exemple comme cela a été détaillé ci-dessus et, alors que le gel de silicone n'est pas encore réticulé, on projette des fines particules d'agent gélifiant sur la surface du gel de silicone. Dans ce mode de réalisation de l'invention, la quantité d'agent gélifiant peut être inférieure à celle utilisée lorsque l'agent gélifiant a été incorporé dans le gel de silicone avant enduction sur le filet.

De plus, ce mode de réalisation de l'invention permet d'obtenir une interface dont la face destinée à être exposée au contact direct de la plaie a reçu par projections un agent gélifiant de telle sorte que seule cette face de l'interface présente un caractère hydrophile, le reste de l'interface demeurant hydrophobe.

C'est pourquoi, selon cette variante de l'invention, le poids d'agent gélifiant peut représenter entre environ 0,1% et 1% du poids du mélange. Cette quantité sera suffisante pour que l'interface selon l'invention présente un caractère amphiphile en surface qui est satisfaisant pour créer l'humidité bienfaitrice pour la cicatrisation de la plaie mentionnée ci-dessus.

Ensuite, on soumet le filet enduit du mélange à une étape de réticulation de manière à ce que le gel de silicone que comprend essentiellement le mélange réticule. On obtient alors une interface selon la présente invention.

Les paramètres de la réticulation du gel de silicone sont parfaitement à la portée de l'homme du métier.

La réticulation est avantageusement effectuée selon un gradient de températures approprié dans un four. Le four dans lequel est effectuée l'étape de réticulation est parfaitement à la portée de l'homme du métier.

Par exemple, un gradient de températures peut être celui qui est détaillé dans le tableau 1 ci-dessous :

**Tableau 1 : gradient de températures au cours de l'étape de réticulation du mélange dont est enduit le filet**

| | | | | | | |
|---|---|---|---|---|---|---|
| Temps (en secondes) | 0 | 15 | 30 | 60 | 90 | 120 |
| Température de réticulation (°C) | 20 | 40- 60 | 80-90 | 120-130 | 130-140 | 140 |

Dans un mode de réalisation de l'invention, l'étape de réticulation du mélange qui comprend essentiellement du gel de silicone est réalisée en environ 2 minutes à une température de 120°C.

Il est à noter que lorsque l'interface est destinée à recouvrir un matériau absorbant comme cela est décrit ci-après, il est aussi envisageable dans le cadre de la présente invention d'appliquer le filet enduit du mélange sur le matériau absorbant, puis de soumettre l'ensemble ainsi constitué par l'interface et le matériau absorbant à une étape de réticulation, et ce de la même façon que cela a été décrit ci-dessus.

De cette manière, on obtient une liaison résistante entre le filet enduit du mélange et le matériau absorbant. En effet, dans ce mode de réalisation de l'invention, le mélange qui comprend essentiellement du gel de silicone va pénétrer dans le matériau absorbant et être absorbé par celui-ci avant que la réticulation du gel de silicone ne soit achevée. De cette façon, l'interface ainsi obtenue sera fixée au matériau absorbant de manière plus cohésive que si elle avait été fixée au matériau absorbant après l'achèvement de la réticulation du gel de silicone.

Lorsque le filet se présente sous la forme d'une bande, à l'issue de la réticulation, il est découpé en interfaces individuelles suivant des dimensions adaptées à l'utilisation de l'interface. L'interface peut être conditionnée en sachets étanches et stérilisés.

La présente invention a aussi pour objet une compresse qui comprend l'interface telle que décrite ci-dessus.

Dans un mode de réalisation de l'invention, ladite compresse comprend au moins une interface telle que décrite ci-dessus et au moins un matériau absorbant, ladite interface étant fixée sur le matériau absorbant. Ainsi, l'ensemble constitué par l'interface et le matériau absorbant forme la compresse.

Dans un mode de réalisation le matériau absorbant peut être choisi parmi :
- les mousses de polyuréthane, et plus préférentiellement les mousses de polyuréthane aliphatique,
- les non tissés tels que les non tissés en viscose ou à base d'un mélange de polyester et de viscose (par exemple, un mélange comprenant en poids de 70% à 80% de viscose et de 20 à 30% de polyester), ou encore les non tissés contenant de la cellulose,
- les matériaux en cellulose,
- les compresses non tissées formées à partir de fibres, par exemple des fibres superabsorbantes telles que des fibres en polyacrylate.

Le choix du matériau absorbant est parfaitement à la portée de l'homme du métier.

La mousse de polyuréthane aliphatique présente l'avantage d'être une mousse absorbante souple à cellules ouvertes qui permet un excellent niveau de rétention d'environ 65 %. Elle présente également des capacités d'absorption de plus de 15 fois son poids. Son épaisseur peut varier de 1,5 à 5 mm. De plus, elle présente l'avantage de résister à l'irradiation des rayons bêta et gamma. Cela est particulièrement approprié lorsque la compresse selon l'invention est stérilisable ; ce qui implique que tous ses constituants doivent être stérilisables.

Ainsi, pendant l'exsudation de la plaie, il y a absorption des exsudats par le matériau absorbant à travers les mailles du filet qui a été enduit d'un mélange qui comprend essentiellement du gel de silicone.

Comme cela a été expliqué ci-dessus, la compresse selon l'invention peut être fabriquée en fixant par collage une interface selon l'invention sur un matériau absorbant. Autrement dit, on fixe sur un matériau absorbant un filet à mailles ouvertes qui a été enduit d'un mélange comprenant au moins un gel de silicone et au moins un agent gélifiant. Le gel de silicone est sous forme réticulée.

Dans un autre mode de réalisation de l'invention, la compresse selon l'invention est fabriquée en disposant un filet à mailles ouvertes tout juste enduit dudit mélange comprenant au moins un gel de silicone et au moins un agent gélifiant sur un matériau absorbant, puis en soumettant l'ensemble comprenant le filet enduit du mélange et le matériau absorbant à une étape de réticulation du gel de silicone. On obtient alors une compresse plus cohésive que selon l'autre mode de fabrication de la compresse selon l'invention dans lequel l'interface selon l'invention est fixée (par exemple par collage) sur un matériau absorbant après la réticulation de gel de silicone.

L'invention a aussi pour objet un pansement comportant au moins une compresse selon l'invention qui est collée sur un support, ledit support présente au moins une zone adhésive configurée pour fixer ledit pansement sur une peau, et notamment au niveau des zones périphériques d'une plaie.

De manière préférée, le pansement selon l'invention est stérilisable. Cela signifie que tous ses constituants sont stérilisables. Cette propriété de stérilisation est indispensable si le pansement selon l'invention est destiné à être appliqué sur des plaies aiguës, chroniques et exsudatives. Ces plaies sont souvent présentes sur des personnes affaiblies (par exemple des grands brûlés ou des personnes en fin de vie). La stérilisation du pansement est alors indispensable pour éviter tous risques de contamination.

Dans un mode de réalisation du pansement selon l'invention, une couche d'un matériau superabsorbant est intercalée entre le matériau absorbant de la compresse et le support qui comporte la zone adhésive. Cela procure l'avantage de renforcer la capacité de rétention des exsudats du pansement selon l'invention.

De manière préférée, la compresse est centrée sur le support du pansement.

Dans un mode de réalisation du pansement selon l'invention, la compresse est disposée de manière centrée sur un support hypoallergénique qui comporte au moins une zone adhésive qui est une couche de colle acrylique ou de gel de silicone, et ce de manière à maintenir en place sur le patient le pansement selon l'invention.

Lorsque la zone adhésive est une couche de gel de silicone, cela permet un retrait atraumatique du pansement selon l'invention.

Selon un mode de réalisation du pansement, le support comprend un film de polyuréthane qui est recouvert d'une couche de non tissé, la couche de non tissé étant elle-même recouverte d'une couche de colle acrylique ou de gel de silicone. Le matériau absorbant de la compresse est fixé sur cette couche de colle acrylique ou de gel de silicone, de préférence de manière centrée. Le matériau absorbant est recouvert de l'interface selon l'invention. Par exemple, l'interface selon l'invention a été fixée (notamment par collage) sur ledit matériau absorbant. Selon une variante de ce mode de réalisation de l'invention, une couche de matériau super-absorbant a été intercalée entre la couche de colle acrylique ou de gel de silicone et le matériau absorbant.

La structure du support telle que décrite ci-dessus procure l'avantage de maintenir correctement en place l'interface selon l'invention sur la plaie, et ce même si le patient prend une douche. En effet, le film de polyuréthane dans la structure du support telle que décrite ci-dessus est avantageux du fait qu'il permet une imperméabilité à l'eau du pansement tout en conservant une très bonne perméabilité à la vapeur d'eau et une excellente conformabilité du pansement selon l'invention.

En outre, avec une telle structure du support, le pansement selon l'invention peut aisément être mis en place par le personnel soignant ou le patient lui-même sur la plaie à cicatriser.

Dans le mode de réalisation du pansement selon l'invention aussi envisageable dans lequel le support est dépourvu de film de polyuréthane et comprend, par exemple, une couche de non tissé recouverte d'une couche d'adhésif, ce pansement ne présentera pas une aussi bonne tenue sous la douche que le pansement décrit ci-dessus avec un film de polyuréthane dans la structure de son support.

Dans un autre mode de réalisation de l'invention, le pansement selon l'invention comprend au moins une couche de matériau qui est imprégnée de principes actifs antiseptiques tels que la chlorhexidine et/ou des sels d'argent.

Dans un mode de réalisation de l'invention, le matériau absorbant de la compresse a été imprégné de ces principes actifs antiseptiques.

Dans un autre mode de réalisation de l'invention, ces principes actifs antiseptiques ont été incorporés dans le mélange qui comprend essentiellement du gel de silicone décrit ci-dessus.

Dans un mode de réalisation de l'invention, l'adhésif de la zone adhésive consiste en une colle acrylique ou en un gel de silicone qui permet un retrait atraumatique du pansement.

Le pansement selon l'invention est particulièrement avantageux lorsque la compresse telle que décrite ci-dessus est destinée à être appliquée sur des plaies exsudatives ou très exsudatives.

En effet, le matériau absorbant va absorber les exsudats de la plaie au fur et à mesure de leur apparition.

L'interface que comprend le pansement selon l'invention est :
- anti-adhérente du fait que le filet a été enduit d'un mélange qui comprend essentiellement du gel de silicone,
- perméable aux exsudats de la plaie du fait qu'elle comprend un filet à mailles ouvertes non obstruées par l'enduction (c'est-à-dire conservation d'une ouverture entre chacune des mailles), et ce en évitant tout contact direct de la plaie avec le matériau absorbant.

Dans un mode réalisation de l'invention, le pansement comprend en outre au moins un film de protection qui est agencé pour recouvrir l'interface et la zone adhésive.

Le matériau du film de protection peut être un polyester (par exemple un poly(téréphtalate d'éthylène)) enduit d'une fine couche de fluorosilicone ou bien un polyéthylène, de préférence un film de polyéthylène gaufré qui est compatible avec le mélange dont est enduit le filet et avec l'adhésif de la zone adhésive du support. Dans un mode de réalisation de l'invention, le matériau du film de protection est un poly(téréphtalate d'éthylène) enduit d'une couche de fluorosilicone.

Il est avantageux d'utiliser un film de polyéthylène, car ce polymère par nature adhère peu au gel de silicone. De manière préférée, on utilise des films embossés et/ou gaufrés afin de réduire les surfaces de contact et ainsi permettre le retrait de ce film aisément de l'interface. Les films de polyéthylène gaufrés pointe de diamant de la société RKW, 55 µm et 65 µm sont préférés.

Ainsi, le matériau du film de protection est choisi de manière appropriée pour être anti-adhérent par rapport au gel de silicone de l'interface.

Dans un mode de réalisation de l'invention, le film de protection tel que décrit ci-dessus est déposé sur le filet de l'interface enduit du mélange qui comprend essentiellement du gel de silicone, et ce juste après l'étape de réticulation mentionnée ci-dessus qui est avantageusement réalisée dans un four. Par exemple, à la sortie du four dans lequel a été effectuée l'étape de réticulation, on dépose un film de polyéthylène gaufré sur le filet enduit du mélange. Le film de protection accroche ainsi légèrement sur le mélange dont est enduit le filet et est entraîné par celui-ci dans la ligne de production de l'interface selon l'invention. Il n'est pas nécessaire d'appliquer une pression sur le film de protection.

Selon un mode de réalisation de l'invention, le pansement comprend en outre un emballage dans lequel il est disposé avant son utilisation, ledit emballage comprend par exemple deux films scellables à chaud et qui sont stérilisables par rayonnement ou par oxyde d'éthylène ou par tout autre moyen de stérilisation des produits médicaux et pharmaceutiques à la portée de l'homme du métier.

D'autres caractéristiques et avantages de l'invention apparaîtront au cours de la description qui va suivre du pansement selon l'invention, donnée à titre d'exemple non limitatif, en référence au dessin annexé dans lequel :

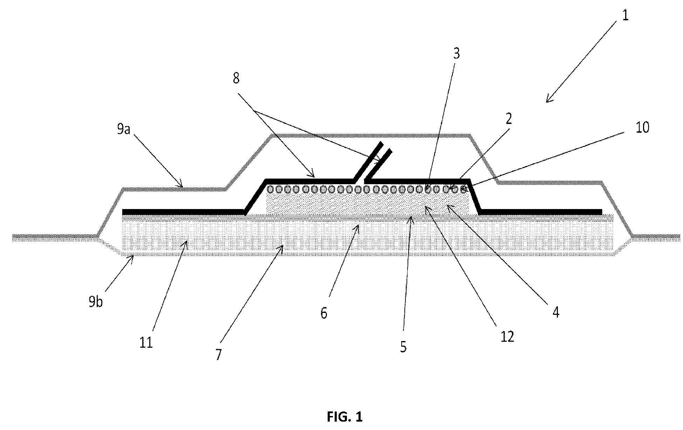

- la figure 1 est une vue schématique selon une coupe longitudinale d'un pansement selon l'invention.

La figure 1 représente une coupe longitudinale d'un pansement 1 selon l'invention. Le pansement 1 comprend une interface 10. Ladite interface 10 comprend un filet 2 en polyester qui a été enduit d'un mélange 3 qui comprend en poids 89% de gel de silicone, 1% d'acide hyaluronique et 10% de CMC. Le filet recouvre un matériau absorbant 4 qui est une mousse de polyuréthane.

L'ensemble constitué par l'interface 10 et le matériau absorbant 4 est une compresse 12 selon l'invention qui est disposée de manière centrée sur un support 11. Plus précisément, le support 11 comprend un film de polyuréthane 7 qui est recouvert d'une couche de non tissé 6. La couche de non tissé 6 est recouverte d'une couche de colle acrylique 5 qui est hypoallergénique.

Deux films de protection 8 en polyéthylène gaufré recouvrent la compresse 12, ainsi que la zone de la couche de colle acrylique 5 qui n'est pas recouverte par la compresse 12.

Le pansement 1 est emballé de manière scellée dans un emballage étanche qui comporte :
- une première face 9a transparente en un matériau comprenant un mélange de polyester, polyéthylène et de poly(téréphtalate) d'éthylène (ci-après abrégé « PET), et
- une seconde face 9b transparente en un matériau comprenant un mélange de PET aluminisé, de polyester métallisé et de polyéthylène sur laquelle repose le film en polyuréthane 7.

### PARTIE EXPERIMENTALE :

La diminution de l'adhérence d'une interface selon l'invention en fonction de la formation du gel a été testée.

Pour ce faire, on a mesuré la force de pelage à 180° sur une plaque en acier d'une interface selon l'invention, et ce en fonction de son hydratation.

Plus précisément, l'interface utilisée au cours de ces tests mesurait 4 cm de largeur et 10 cm de longueur et comprenait un filet en polyéthylène qui avait été enduit d'un mélange comprenant en poids 90% de gel de silicone et 10% de CMC, selon un grammage de 200 g/m².

Le poids d'eau, exprimé en grammes, qui a été déposé sur l'interface est détaillé dans le tableau 2 ci-dessous.

Chaque interface ainsi hydratée a été collée sur la plaque en acier.

On a mesuré avec un dynamomètre selon la norme NF EN 1943 la force nécessaire au décollement de ladite interface hydratée de la plaque en acier.

En outre, on a observé la formation de gel à la surface de l'interface. Le pourcentage de la surface de l'interface sur laquelle on a observé la formation de gel est détaillé dans le tableau 2 ci-dessous.

**Tableau 2 détaillant la force de pelage et le pourcentage de gel formé et observé à la surface de l'interface en fonction du poids d'eau déposée sur l'interface**

| **Poids d'eau déposée en g** | **0** | **0,25** | **0,5** | **0,75** |
|---|---|---|---|---|
| **Force de pelage mesurée en newtons (N)** | Entre 0,60 et 0,80 | Entre 0 et 0,80 | Entre 0 et 0,60 | 0 |
| **Surface de gel en %** | 0 | 30% | 65% | 100% |

Au vu du tableau 2, on constate ainsi une baisse du pouvoir adhésif de l'interface selon l'invention avec la formation du gel qui est aussi proportionnelle à l'eau absorbée.

On constate qu'à partir d'une absorption de 0,75 g d'eau sur 40 cm² d'une interface selon l'invention, il n'est plus possible de mesurer la moindre force adhésive au décollement tangentiel (autrement dit la force de pelage).

Entre 0,25 g et 0,75 g d'eau absorbée sur 40 cm² d'une interface selon l'invention, la force adhésive (autrement dit la force de pelage) diminue et les mesures sont très « chaotiques » : on observe des zones parfaitement non adhérentes (force de pelage nulle) et quelques points de résistance correspondant à des zones non complètement hydratées où l'on mesure des adhérences faibles comprises entre 0,60 et 0,80 N.

De plus, des tests d'absorption et de rétention ont été menés respectivement sur :
- une 1^{ière} compresse circulaire de 50 mm de diamètre composée de, en pourcentages en poids par rapport au poids total de la dite compresse, 25% de fibres de viscose, 25% de fibres de polyester et de 50% de fibres de polyacrylate et qui comportait en outre une interface selon l'invention telle que décrite juste ci-dessus dans la partie expérimentale, à savoir une compresse selon l'invention, et
- sur 2^{ième} compresse présentant les mêmes caractéristiques que la 1^{ière} compresse mais qui était dépourvue d'une interface selon l'invention, à savoir une compresse comparative.

Les tests ont été réalisés selon le protocole expérimental décrit au chapitre 3.3 intitulé « Capacité de rétention des fluides » de la norme NF EN 13726-1.

La solution utilisée était une solution de chlorure de sodium comprenant 8,66 g et complétée à un litre d'eau. La température de l'enceinte climatique était de à 37°C. Les mesures ont été effectuées après 24 et 48 heures.

Le tableau 3 ci-dessous détaille :
- la masse de fluide absorbé sur la compresse testée après 24 heures et 48 heures,
- la capacité de rétention du fluide sur la compresse testée après 24 heures et 48 heures.

**Tableau 3 détaillant la masse de fluide absorbé et la capacité de rétention de fluide après 24 et 48 heures.**

| | Masse fluide absorbée après 24 h (g/24h) | Capacité de rétention du fluide après 24h (g/24h) | Masse fluide absorbée après 48 h (g/48 h) | Capacités de rétention du fluide après 48 h (g/48h) |
|---|---|---|---|---|
| Compresse selon l'invention | 1,9848 | 3,2284 | 2,0437 | 4,1222 |
| Compresse comparative | 2,0134 | 3,3024 | 2,0909 | 4,2146 |
| Variations de performances | -1,4% | -2,2% | -2,2% | -2,2% |

Les différences entre les capacités d'absorption et de rétention de la compresse selon l'invention et de la compresse comparative ne varient que de 1,4% à 2,2%. Cela n'est pas significatif au vu des aléas de ces mesures.

Ainsi, une interface selon l'invention demeure parfaitement ouverte au cours de son utilisation et peut laisser passer sans obstacle les exsudats d'une plaie de par son excellente ouverture et sa perméabilité. Autrement dit, il n'y a pas obstruction des mailles ouvertes du filet de l'interface selon l'invention au cours de l'utilisation de la compresse selon l'invention.

## Revendications

1. Interface cicatrisante non adhérente (10) comportant au moins un filet (2) à mailles ouvertes, **caractérisée en ce que** le filet (2) a été enduit d'un mélange (3) comprenant au moins du gel de silicone et au moins un agent gélifiant.

2. Interface selon la revendication 1, **caractérisée en ce qu'**au moins une partie desdites mailles ne sont pas obstruées par ledit mélange (3) comprenant au moins du gel de silicone et au moins un agent gélifiant.

3. Interface selon la revendication 1 ou 2, **caractérisée en ce qu'**une fois ledit filet (2) enduit avec le mélange (3) contenant au moins du gel de silicone et l'agent gélifiant, le taux d'ouverture du filet (2) est préférentiellement compris entre 40% et 90 %, de préférence entre 75 % et 90 %.

4. Interface selon l'une quelconque des revendications 1 à 3, **caractérisée en ce que** le taux de perméabilité à la vapeur d'eau de l'interface (10) est compris entre 50 et 90 g/m²/24 heures, de préférence entre 75 et 90 g/m²/24 heures.

5. Interface cicatrisante non adhérente (10) selon l'une quelconque des revendications 1 à 4, **caractérisée en ce que** le poids en gel de silicone représente au moins 80% du poids total dudit mélange (3).

6. Interface cicatrisante non adhérente (10) selon l'une quelconque des revendications 1 à 5, **caractérisée en ce que** le matériau des fils du filet (2) est choisi parmi les polyesters, les polypropylènes, les polyéthylènes, les polyamides et les acétates de cellulose.

7. Interface cicatrisante non adhérente (10) selon l'une quelconque des revendications 1 à 6, **caractérisée en ce que** le pourcentage massique de l'agent gélifiant dans ledit mélange (3) est compris entre 0,1% et 20%.

8. Interface cicatrisante non adhérente (10) selon l'une quelconque des revendications 1 à 7, **caractérisée en ce que** l'agent gélifiant est choisi parmi l'hydroxypropylméthylcellulose, l'hydroxypropyléthylcellulose, la méthylcellulose, la carboxyméthylcellulose, l'hydroxyéthylcellulose, l'éthylcellulose, l'éthylhydroxyethylcellulose, l'hydroxypropylcellulose, les alginates tels que l'alginate de sodium, l'acide alginique, l'acide polyacrylique, la gomme de xanthane, la gomme adragante, la gomme de guar, l'alcool polyvinylique, les polyvinylpyrrolidones, les copolymères des polyoxyéthylènes et des polyoxypropylènes, le chitosane, et les pectines.

9. Interface cicatrisante non adhérente (10) selon l'une quelconque des revendications 1 à 8, **caractérisée en ce que** la quantité du mélange (3) est comprise entre 50 et 300 g/m² de ladite interface (10).

10. Procédé de fabrication d'une interface (10) selon l'une quelconque des revendications 1 à 9, **caractérisé en ce qu'**il comprend au moins les étapes suivantes :
a) On prépare un 1^{ier} mélange préparatoire comprenant au moins un agent gélifiant ;
b) On incorpore ce 1^{ier} mélange préparatoire dans une 1^{ière} composition qui comprend au moins une résine de poly-di-organosiloxane et un catalyseur de manière à obtenir un 2^{ième} mélange préparatoire ;
c) On incorpore dans ce 2^{ième} mélange préparatoire ainsi obtenu une 2^{ième} composition qui comprend au moins la résine de poly-di-organosiloxane et qui est dépourvue de catalyseur;
d) On mélange l'ensemble ainsi obtenu à l'étape c) jusqu'à l'obtention d'un mélange (3) homogène qui comprend essentiellement du gel de silicone ;
e) On enduit le mélange (3) obtenu à l'étape d) sur les fils d'un filet (2) ;
f) On réticule le gel de silicone que comprend ledit mélange (3).

11. Procédé de fabrication d'une interface (10) selon la revendication 10, **caractérisé en ce que** l'étape e) d'enduction du mélange (3) sur les fils du filet (2) comprend au moins les étapes :
a) On trempe intégralement le filet (2) dans ledit mélange (3) de manière à ce que le filet (2) se charge d'un excédent dudit mélange (3) ;
b) On lamine le filet (2) ;
c) On achemine le filet (2) sous un 1^{ier} racle d'enduction qui pré-régule le grammage du mélange (3) déposé sur le filet (2) ;
d) On achemine le filet (2) sous un 2^{ième} racle d'enduction pour réguler finement le grammage final du mélange (3) déposé sur ledit filet (2).

12. Procédé de fabrication d'une interface (10) selon la revendication 10 ou 11, **caractérisé en ce qu'**après l'étape e) d'enduction, on effectue un soufflage d'air sur le filet (2) et/ou on fait passer le filet (2) sur un cylindre en inox qui est configuré pour que le mélange (3) en excédent reste collé sur ledit cylindre en inox.

13. Compresse (12) comprenant au moins une interface (10) selon l'une quelconque des revendications 1 à 9 et au moins un matériau absorbant (4), ladite interface (10) étant fixée sur ledit matériau absorbant (4).

14. Procédé de fabrication d'une compresse (12) selon la revendication 13, caractérisé en en qu'on dispose un filet (2) tout juste enduit du mélange (3) sur un matériau absorbant (4), puis on soumet l'ensemble comprenant le filet (2) enduit du mélange (3) et le matériau absorbant (4) à une étape de réticulation du gel de silicone.

15. Pansement (1) comportant au moins une compresse (12) selon la revendication 13 qui est collée sur un support (11), ledit support (11) présentant au moins une zone adhésive (5) configurée pour fixer ledit pansement (1) sur une peau.
